# EUROPEAN PATENT APPLICATION

(11) **EP 1 671 662 A1**
(43) Date of publication of application: **21.06.2006**
(21) Application number: 03818522.9
(22) Date of filing: 27.08.2003
(51) Int. Cl.: A61L 27/40, A61L 27/42, A61L 27/44

(54) **STRUCTURAL BODY CONSTITUTED OF BIOCOMPATIBLE MATERIAL IMPREGNATED WITH FINE BONE DUST AND PROCESS FOR PRODUCING THE SAME**

(71) Applicant: Ogiso, Makoto, Tokyo 112-0006 (JP); Pentax Corporation, Tokyo 174-8639 (JP)
(72) Inventor: OGISO, Makoto, Bunkyo-ku, Tokyo 112-0006 (JP)
(74) Representative: Schaumburg, Thoenes, Thurn, Landskron
(86) International application number: PCT/JP2003/010826
(87) International publication number: WO 2005/023325

(57) **Abstract**

A porous structure or surface-roughened structure impregnated with fine bone powder is usable as an artificial bone or artificial dental root. The porous structure has communicating macro-pores having an average diameter of 100-1000 µm open on its entire outer surface at a density of 1 or more per an area of 1000 µm x 1000 µm, and fine communicating pores or recesses having an average diameter of 0.005-50 µm open in the communicating macro-pores at a density of 1 or more per an area of 50 µm x 50 µm. The surface-roughened structure has fine recesses having an average diameter of 0.005-50 µm and an average depth of 0.005-50 µm open on its entire outer surface at a density of 1 or more per an area of 50 µm x 50 µm.

## Description

### FIELD OF THE INVENTION

The present invention relates to a porous or surface-roughened structure suitable for artificial bones and dental roots and its production method, particularly to a porous or surface-roughened structure of a biocompatible material impregnated with fine bone powder and its production method.

### BACKGROUND OF THE INVENTION

Bone transplantation is conducted to wide varieties of bone defects caused by the removal of bones due to high-level injury, bone tumors, etc. in human bodies, and the use of an autologous (self) bone is considered best to regenerate bone. However, the amount of bone taken is limited, and larger invasion occurs in a portion, from which a larger amount of bone has been removed.

In place of the autologous bone that should be taken in a large amount, an artificial bone made of a biocompatible material is used. However, because the artificial bone has no bone-inducing capability, its use is limited.

Though an artificial dental root is used as one of the treatment methods after tooth loss, it cannot be used when the absorption or shrinkage of jawbone has proceeded, and a conventional detachable artificial tooth should be used.

For the purpose of bone regeneration, investigations are now conducted to provide a method of applying various cell proliferation factors, which can become bone-inducing factors to portions to be treated, a method of cultivating mesenchymal stem cells in the bone marrow using various factors for differentiation to osteoblasts, and transplanting the osteoblasts to portions to be treated, etc.

From the fact that the transplantation of demineralized bone into the muscle causes bone formation, Urist discovered in the early 1960s that a protein exhibiting ectopic bone-inducing activity was contained in the matrix of bone, kicking off research on the application of cell proliferation factors to portions to be treated. Thereafter, development has led to the purification of various proliferation/differentiation factors contained in bone, which contribute to the formation of bone, the determination of partial sequences of amino acids, gene cloning, etc. Particularly, a group of proliferation factors (BMPs) contributing to the formation of bone were discovered, and typical recombinant BMP-2 (rhBMP-2) was made commercially available. To form bone of about 5 mm x 5 mm x 5 mm in a rat muscle, several tens of micrograms of rhBMP-2 is needed, though it is extremely expensive at present. Though good bone is ectopically formed in mice and rats, good results are not obtained in larger animals, particularly primates such as apes, and there is doubt about the effects on humans [see, for instance, Masaki Noda, Akira Nitoh, Kunikazu Tsuji, "Regeneration of Bone and BMP," Inflammation and Regeneration, 21(4), 425, 2001]. Accordingly, the development of controlled-release materials impregnated with BMP (see, for instance, Yasuhiko Tabata, "Repair of Bone by Cell-Proliferating Factors," Resume of the 23rd Meeting of the Japanese Society for Biomaterials, 48, 2001), and the investigation of other proliferating factors than BMP (see, for instance, Hiroshi Tanaka, et al., "Effects of bFGF (Basic Fibroblast Growth Factor) on Differentiation/Proliferation of Interstitial Cells in Bone Marrow," the Journal of Orthopaedic Science, 72(8), S1498, 1998, and Ken Okazaki, et al., "Expression of Insulin-Like Growth Factor 1 in Formation of Osteophyte in Experimental Arthrosis," the Journal of Orthopaedic Science, 72(8), S1484, 1998) were conducted, with no clinical effectiveness clarified.

Differentiation to osteoblasts for local transplantation is carried out by a method of collecting a bone marrow liquid, proliferating stem cells therein in a culture, differentiating them to osteoblasts, and transplanting their cells [see, for instance, Akihiro Umezawa, "Regeneration of Organs And Transplantation of Cells Using Interstitial Cells in Bone Marrow," Inflammation and Regeneration, 21 (4), 437, 2001], or a tissue engineering method of impregnating or mixing an artificial bone with cells, and transplanting it to a portion to be treated in a human body [see, for instance, Hajime Ohgushi, et al., "Bone Regeneration Using Bone Marrow Stem Cells," Inflammation and Regeneration, 21(4), 434, 2001].

However, there is limitation in the proliferation and differentiation of stem cells to osteoblasts, with a bottleneck of how to extract a large amount of stem cells from the bone marrow liquid. Only a small amount of bone can be formed at present, with limited clinical applications just started. In this method, various proliferating factors and steroid hormones are added to cultured stem cells, leaving doubt about whether or not osteoblasts differentiated in a culture system are the same as in a human body. It has recently been reported that the fusion of adult stem cells with cells in an existing tissue may form genetically hodgepodge cells, casting doubt about the clinical application of adult stem cells [see, for instance, N. Terada, et al., "Bone Marrow Cells Adopt Phenotype of Other Cells by Spontaneous Cell Fusion," *Nature* 416, 542-545 (2002), Ying, Q-L., et al., "Changing Potency by Spontaneous Fusion," *Nature* 416, 545-548 (2002)]. Further, when animal (cow) serum is used as a culture liquid, there is a risk of unknown infection.

In artificial bones and dental roots such as outer heads, etc. implanted in the bone, it is desired that they be integrated with a surrounding bone and strongly regenerated as soon as possible after the implantation. Attempts were conducted to provide artificial bones and dental roots with rough surface or make them porous [see, for instance, Hall, J., et al., "Properties of New Porous Oxide Surface on Titanium Implants," Applied Osseointegration Research, 1 (1), 5-8 (2000), Wieland, M., et al., "Wavelength-Dependent Roughness: A Quantitative Approach to Characterizing The Topography of Rough Titanium Surfaces," Int. J. Oral Maxillofac Implants, 16 (2), 163-181 (2001)]. These attempts are effective when the surrounding bone has high osteoblastic (bone-forming) activity after the implantation of artificial bones and dental roots. However, when the surrounding bone has low activity, the effects of these methods are dubious [see, for instance, Ogiso, M., et al., "The Delay Method: A New Surgical Technique for Enhancing The Bone-Binding Capability of HAP Implants to Bone Surrounding Implant Cavity Preparations," J. Biomed. Mater. Res., 18, 805-812 (1994)].

As described above, the technique of medically repairing large bone defects has not been established yet. In the case of using artificial dental roots in patients with advanced absorption and shrinkage of jawbone, too, a bone regeneration technique needing no living bone in a large amount is desired. Further, in the implantation of artificial bones and dental roots such as outer heads, etc. in the bone, they are required to be strongly integrated with the surrounding bone as soon as possible after the implantation.

### OBJECT OF THE INVENTION

Accordingly, an object of the present invention is to provide a bone-powder-impregnated, porous or surface-roughened structure made of a biocompatible material, which is clinically suitable for artificial bones and dental roots capable of inducing spontaneous formation of bone, and its production method.

### DISCLOSURE OF THE INVENTION

The bone-powder-impregnated, porous structure of the present invention comprises a porous matrix made of a biocompatible material impregnated with fine bone powder. The bone-powder-impregnated, porous structure of the present invention includes the following structures.

The first bone-powder-impregnated, porous structure has fine communicating pores having an average diameter of 0.005-50 µm in its entire body, the fine communicating pores being open on an outer surface of said porous structure at a density of 1 or more per an area of 50 µm x 50 µm.

The second bone-powder-impregnated, porous structure has communicating macro-pores having an average diameter of 100-1000 µm in its entire body, which are open on an outer surface of said porous structure at a density of 1 or more per an area of 1000 µm x 1000 µm, and fine communicating pores having an average diameter of 0.005-50 µm, which are open on inner walls of said communicating macro-pores at a density of 1 or more per an area of 50 µm x 50 µm.

The third bone-powder-impregnated, porous structure has communicating macro-pores having an average diameter of 100-1000 µm in its entire body, which are open on an outer surface of said porous structure at a density of 1 or more per an area of 1000 µm x 1000 µm, and fine recesses having an average diameter of 0.005-50 µm and an average depth of 0.005-50 µm, which are open on inner walls of said communicating macro-pores at a density of 1 or more per an area of 50 µm x 50 µm.

The bone-powder-impregnated, porous structure of the present invention is porous in the entire structure or only in a surface layer of said structure.

The bone-powder-impregnated, surface-roughened structure of the present invention comprises a surface-roughened matrix made of a biocompatible material, which is impregnated with fine bone powder.

In the bone-powder-impregnated, surface-roughened structure according to a preferred embodiment of the present invention, the surface-roughened structure has fine recesses having an average diameter of 0.005-50 µm and an average depth of 0.005-50 µm, which are open on its entire outer surface at a density of 1 or more per an area of 50 µm x 50 µm.

The method for producing a bone-powder-impregnated, porous structure (or surface-roughened structure) according to the present invention comprises the steps of preparing fine bone powder, and impregnating the porous structure (or surface-roughened structure) with the fine bone powder. The porous structure (or a rough surface of the surface-roughened structure) is preferably impregnated with fine bone powder in the form of a suspension.

The artificial bone according to a preferred embodiment of the present invention comprises a porous structure impregnated with bone powder. The artificial bone or dental root according to another preferred embodiment of the present invention is constituted by a structure with a porous surface layer, which is impregnated with bone powder. The artificial bone or dental root according to a further embodiment of the present invention is constituted by a surface-roughened structure, which is impregnated with bone powder.

The biocompatible material constituting the above porous or surface-roughened structure is preferably at least one selected from the group consisting of ceramics, metals, and polymers. The ceramics are preferably calcium phosphate ceramics.

The fine bone powder impregnating the above porous or surface-roughened structure is preferably pulverized living bone or demineralized bone powder. The fine bone powder preferably has an average diameter of 50 µm or less.

### BRIEF DESCRIPTION OF THE DRAWINGS

Fig. 1 is a photomicrograph (magnification: 200 times) showing bone formed on surfaces of the communicating macro-pores of the bone-powder-impregnated, porous structure in Example 1 at a time when 4 weeks passed from the implantation.
Fig. 2 is a photomicrograph (magnification: 16 times) showing bone formed in the entire bone-powder-impregnated, porous structure in Example 1 at a time when 8 weeks passed from the implantation.
Fig. 3 is an electron photomicrograph (magnification: 430 times) showing a surface state of the surface-roughened artificial dental root of hydroxyapatite used in Example 2.
Fig. 4 is an electron photomicrograph (magnification: 6200 times) showing recesses formed between primary hydroxyapatite particles on a surface of the surface-roughened artificial dental root of hydroxyapatite used in Example 2.
Fig. 5 is a photomicrograph (magnification: 40 times) showing bone formed on an entire surface of the bone-powder-impregnated, surface-roughened, artificial dental root of hydroxyapatite in Example 2 at a time when 4 weeks passed from the implantation.
Fig. 6 is a photomicrograph (magnification: 200 times) showing that bone formed on a surface of the bone-powder-impregnated, surface-roughened, artificial dental root of hydroxyapatite was directly bonded to fine raggedness of the artificial dental root in Example 2 at a time when 4 weeks passed from the implantation.
Fig. 7 is a photomicrograph (magnification: 53 times) showing bone formed in a wide region on the bone-powder-impregnated, surface-porous, artificial dental root of titanium in Example 3 at a time when 4 weeks passed from the implantation.
Fig. 8 is a photomicrograph (magnification: 200 times) showing that there was no uncalcified layer between a porous surface layer of TiO₂ on the bone-powder-impregnated, surface-porous, artificial dental root of titanium and bone formed on the porous surface layer in Example 3, at a time when 4 weeks passed from the implantation.

### THE BEST MODE FOR CARRYING OUT THE INVENTION

Remodeling repeated in healthy animal bone usually causes the coupling of absorption and regeneration in bone. However, the absorption and regeneration of bone are not always balanced.

In the case of bone fracture, a large amount of bone is formed in an injured portion, with slight bone absorption occurring. When a bone is bored, the formation of bone occurs so actively as to fill the hole while only slight bone absorption occurs in a cut surface of the bone. Also, when yellow bone marrow is washed away in a cancellous bone region, a large amount of bone is formed in that bone marrow region, while bone absorption occurs only slightly on the original bone surface, which is a center of forming bone. These facts indicate that the bone inherently has the ability to regenerate bone more than absorbed, namely an excess self-regenerating function.

The bone matrix contains various proliferation factors contributing to the formation of bone. BMP is a proliferating factor, which is considered to function in an early stage of a bone-forming process, but as described above, it is difficult to form bone in humans simply by giving BMP alone. It is presumed that the regeneration of bone more than absorbed as described above starts, when various bone-proliferating factors including BMP are freed from the bone and activated by bone absorption, and act on mesenchymal stem cells proliferated in an injured portion compositely and efficiently to provide a bone-regenerating environment around the bone-absorbed portion. The formation of bone in such environment starts when osteoblasts are formed by differentiation on and near a bone surface on which bone absorption occurs. Thus, osteoblasts usually are not formed spontaneously in portions separate from the bone.

The mesenchymal stem cells differentiatable to osteoblasts do not always exist only in a bone tissue. Undifferentiated mesenchymal cells or mesenchymal stem cells differentiatable to various cells including osteoblasts remain in soft tissues such as connective tissues, muscles, etc. including fat tissues obtained from the mesenchymal tissue, which is an embryological origin of bone.

The absorption and regeneration of bone is balanced in the remodeling of healthy bone, so that no more bone than necessary is regenerated, and no bone is usually formed in fat tissues and muscles, presumably because of the lack of a bone morphogenetic protein (bone-forming factor) or the function of a bone morphogenesis-preventing factor.

The above facts suggest that a large amount of bone can be formed not only in bone tissues but also in fat tissues, fibrous connective tissues and muscle tissues, if bone containing the bone-forming factor is well used in an artificial space where the bone morphogenesis-preventing factor can be excluded. In the course of bone formation, it is necessary to meet the conditions that the space becomes rich in the bone-proliferating factor freed from the bone, and that part of the bone remains.

Accordingly, the present invention provides a bone-powder-impregnated structure (porous or surface-roughened structure) comprising a biocompatible material capable of regenerating a large amount of bone from a small amount of bone, by setting an environment in which the maximum bone-regenerating function can be exhibited. Specifically, the above bone-powder-impregnated structure for proliferating bone may be (a) a porous structure comprising a biocompatible material having fine communicating pores open at a surface, which are impregnated with a small amount of fine bone powder, (b) a porous structure comprising a biocompatible material having communicating macro-pores open at a surface and fine communicating pores open in the communicating macro-pores, which are impregnated with a small amount of fine bone powder, (c) a porous structure comprising a biocompatible material having communicating macro-pores open at a surface and fine recesses open in the communicating macro-pores, which are impregnated with a small amount of fine bone powder, or (d) a surface-roughened structure comprising a biocompatible material having fine recesses open at a surface, which are impregnated with a small amount of fine bone powder. These bone-powder-impregnated structures are implanted in a soft tissue or bone of a human body, or in a human body such that it is in contact with a bone.

In the present invention, the bone powder is efficiently absorbed by osteoclasts and macrophage in the communicating macro-pores as quickly as possible after the implantation, to create an environment rich in the bone-proliferating factor necessary for bone formation in the porous structure, and to cause bone powder in the fine communicating pores or the recesses to avoid the attack of osteoclasts and macrophage, thereby inducing the differentiation of mesenchymal stem cells proliferated in the communicating macro-pores to osteoblasts. Namely, a composite comprising a biocompatible material having fine communicating pores or recesses and fine bone powder received in the communicating pores or recesses acts as a scaffold for differentiation to osteoblasts. By causing these phenomena in the porous structure, the influence of a bone morphogenesis-preventing factor that is considered to exist in tissues outside the porous structure is excluded, making it possible to form a large amount of bone in the porous structure.

In an artificial bone or dental root such as an outer head, etc. implanted in a bone, it is desirably fused to the surrounding bone as soon as possible after the implantation. The most effective means therefor is not to wait for the bone formation starting from the surrounding bone tissue or the cut surface of the bone reaches the artificial bone or dental root so that the bone being formed is bonded to the artificial bone or dental root, but to provide the artificial bone or dental root with spontaneous bone-forming capability. When an artificial bone or dental root having no spontaneous bone-forming capability is implanted in a bone having low bone-forming activity, a fibrous connective tissue is formed around the artificial bone or dental root before bone formation reaches from a living bone to the implanted artificial bone or dental root, resulting in difficulty in binding bone [see Ogiso, M., et al., "The Delay Method: A New Surgical Technique For Enhancing The Bone-Binding Capability of Hap Implants To Bone Surrounding Implantation Cavity Preparations," J. Biomed. Mater. Res., 18, 805-812 (1994)].

However, when the artificial bone or dental root is provided with a porous or rough surface to have fine communicating pores or recesses impregnated with fine bone powder as a scaffold on which osteoblasts are formed by differentiation, the artificial bone or dental root can be provided with spontaneous bone-forming capability, thereby preventing a fibrous connective tissue from surrounding it. To form a thick bone even on the artificial bone or dental root, a thick, porous layer having communicating macro-pores open at a surface and fine communicating pores open in the communicating macro-pores or recesses is formed on the artificial bone or dental root, the porous layer being impregnated with fine bone powder.

What induces bone formation is not limited to the bone. The dentin and cement constituting most of teeth, which are embryologically close to the bone and have resembling tissue structures, are also effective as bone-forming materials. In fact, it is reported that demineralized tooth roots have osteoinductive property [see Monica, F. G., et al., "Histologic Evaluation of The Osteoinductive Property of Autogeneous Demineralized Dentin Matrix on Surgical Bone Defects in Rabbit Skulls Using Human Amniotic Membrane for Guided Bone Regeneration," Int. J. Maxillofac Implants, 16, 563-571 (2001)] . Accordingly, biomaterials for artificial bones or dental roots may be impregnated with pulverized tooth powder in place of pulverized bone powder in the present invention.

The bone-powder-impregnated, porous or surface-roughened structure of the present invention and its production method will be explained in detail below.

### [1] Composition

### (A) Matrix of structure

The porous or surface-roughened structure is made of a biocompatible material, which may be any one of ceramics, polymers and metals.

The ceramics may be calcium phosphate ceramics such as hydroxyapatite and calcium triphosphate, alumina, zirconia, burned bones of animals, etc., though not restrictive.

The polymers may be biodegradable polymers based on chitin and chitosan, collagen and gelatin, polylactic acid, polyglycolic acid, etc., and polymers based on methyl methacrylate, 2-hydroxyethyl methacrylate, etc., though not restrictive.

The metals may be titanium, titanium alloys, etc., though not restrictive.

### (B) Bone powder

The bone powder for impregnating the porous or surface-roughened structure may be formed from bones or teeth. Accordingly, the term "bone powder" includes not only pulverized bone powder but also pulverized tooth powder:

### (1) Bones

The bones include not only human bones, but also bones of wide varieties of vertebrates such as mammals (cow, etc.), fish, etc. The collected bone is preferably treated to have weakened antigenicity. Among them, the human bone is preferable, and the autologous bone is particularly preferable. The human bone is commercially available in the U.S., etc.

Portions from which the bone is collected may be, for instance, ilium, jawbone, tibia, and femur, though not particularly restrictive.

In the case of usual autologous bone transplantation, the cancellous bone containing a high-activity bone marrow or the bone containing the cancellous bone is preferable, and the cortex bone is not suitable for transplantation because it contains small amounts of cell components. However, the present invention may use not only the cancellous bone but also the cortex bone.

The bone to be pulverized may include not only a living one but also a chemically treated one, such as demineralized bone, which is an acid-treated bone. When bones other than the autologous bone are used, it is considered that good transplantation results can be obtained, because demineralization reduces antigenicity and activates differentiation/proliferation factors in the bone matrix. Specifically, the demineralization is conducted at a temperature of 4°C using hydrochloric acid at a concentration of about 0.5-0.6 M. Though the demineralization time may differ depending on the size and amount of bone pieces, it takes about 2 days to completely demineralize 1 kg of bone powder having a diameter of 0.5 mm, when 20 L of hydrochloric acid in total is used while well stirring by once exchanging 10 L of hydrochloric acid. After the demineralization, the bone is washed with a large amount of a physiological saline solution, or a large amount of running water until the pH becomes neutral, and finally neutralized by washing with distilled water. With water replaced with acetone, the bone is preferably deprived of fats and then dried. Accordingly, the term "bone powder" includes not only living bone powder but also chemically treated bone powder (for instance, demineralized bone powder, etc.).

Both of the living bone powder and the chemically treated bone powder are preferably stored in a freezer. An ethylene oxide gas may be used to sterilize the bone powder.

### (2) Teeth

Any teeth may be used as long as they are animal teeth having dentin.

### (3) Preparation of bone powder

Using a surgical knife, a trepan bar, etc., bone pieces are taken from a human bone. The teeth are removed from the jaw by an elevator, forceps, etc. Pulverization is carried out by using a mortar, a pulverizer, etc. A container for the mortar and the pulverizer is made of a harder material than hydroxyapatite, an inorganic component of the bone and the tooth. Before pulverization, they are preferably finely disintegrated by pliers, etc. When using a pulverizer, the pulverization can be efficiently conducted if the container is cooled, and the bone or fine tooth pieces are frozen.

The bone powder has such size that it can easily impregnate communicating macro-pores of the porous structure, and that part of the bone powder can enter into fine communicating pores or recesses of the porous structure or fine recesses of the surface-roughened structure. To produce a large amount of bone powder from a small amount of bone, the bone powder particles should be extremely small. Accordingly, the average diameter of the bone powder is 50 µm or less, preferably 20 µm or less including sub-micron particles. The average diameter of the bone powder can be determined by image-processing a photomicrograph of the bone powder, automatically measuring the size of each bone powder, and dividing a sum of the sizes of the bone powder particles by their number, though not restrictive. The average diameter of the following granules, etc. can also be determined by the same method.

A small amount of bone or tooth is pulverized to the above average diameter, to obtain bone powder that can be suspended in fine communicating pores or recesses at a high density in a wide region. When the chemically-treated, demineralized bone is pulverized, fine pulverization is preferably carried out after the demineralization of large bone.

### [2] Structure of porous structure

The porous structure used in the present invention is not restricted to an entirely porous structure, but includes a porous structure, only a surface layer of which is porous. In any case, the porosity of the porous structure may be properly selected depending on applications. For instance, the porosity is preferably 60-95% for high-porosity applications, and 40-60% for high-strength applications.

### (A) Entirely porous structure

The entirely porous structure useful for artificial bones for bone regeneration includes (1) a relatively large porous block for inducing bone formation in its entire body, and (2) a relatively small, granular, porous structure embedded in bone defects or recesses to induce bone formation in the embedded region.

### (1) Porous block

The porous block includes (a) a porous structure having communicating macro-pores having an average diameter of 100-1000 µm, preferably 100-500 µm, which are open on its entire outer surface at a density of 1 or more per an area of 1000 µm x 1000 µm, and fine communicating pores having an average diameter of 0.005-50 µm, which are open in the communicating macro-pores at a density of 1 or more per an area of 50 µm x 50 µm, and (b) a porous structure having communicating macro-pores having an average diameter of 100-1000 µm, preferably 100-500 µm, which are open on its entire outer surface at a density of 1 or more per an area of 1000 µm x 1000 µm, and fine recesses having an average diameter of 0.005-50 µm and an average depth of 0.005-50 µm, which are open in the communicating macro-pores at a density of 1 or more per an area of 50 µm x 50 µm.

The communicating macro-pores are constituted by pluralities of communicating pores open on the entire outer surface of the porous structure. Though the porous structure may have isolated pores, their percentage is preferably as small as possible, because the isolated pores do not contribute to the impregnation of fine bone powder. In the porous sintered bodies of ceramics or metals and foamed polymers, it is clear that most pores are communicating because of their production methods. At least ends of pluralities of fine communicating pores existing in biocompatible material portions between the communicating macro-pores are open to the communicating macro-pores. The fine recesses open in the communicating macro-pores are as relatively shallow as 50 µm or less in depth and exist in the biocompatible material portions between the communicating macro-pores, with their ends open to the communicating macro-pores.

When the communicating macro-pores have pore sizes of less than 100 µm, the intrusion of blood vessels deep into the porous structure is slow, failing to form bone well in the porous structure even though it is impregnated with bone powder. When the porous structure is as thick as 5 mm or more, the communicating macro-pores preferably have pore sizes of more than 100 µm.

On the other hand, when the communicating macro-pores have pore sizes of more than 1000 µm, the amount of bone formed in the porous structure is substantially reduced, because the formation of bone in the porous structure occurs mainly on surfaces of the communicating macro-pores, while bone marrow is formed in center portions of the communicating macro-pores. In addition, when the communicating macro-pores have pore sizes of more than 1000 µm, bone morphogenesis-preventing factors in the surrounding soft tissues are likely to exert influence on the inside of the porous structure. Accordingly, the upper limit of the pore sizes of the communicating macro-pores is 1000 µm. When the porous structure is as thick as 10 mm or less, the communicating macro-pores preferably have pore sizes of 500 µm or less.

The fine communicating pores open in the communicating macro-pores or recesses are preferably as small as 50 µm or less, because it is important that the fine communicating pores or recesses are impregnated with fine bone powder, and that the bone powder partially remains even after attacked by osteoclasts and macrophage. However, when the fine communicating pores or recesses are as small as less than 0.005 µm, they cannot be impregnated with bone crystals, the smallest bone bodies, and cell processes cannot enter into them, failing to cause the differentiation of osteoblasts.

It should be understood that the term "fine communicating pores open in the communicating macro-pores or recesses " means that the fine communicating pores or recesses are not only open in the communicating macro-pores, but part of the fine communicating pores or recesses may be open to the outer surface of said porous structure. In fact, some commercially available porous structures have fine communicating pores or recesses open at outer surfaces. Because the total surface area of the communicating macro-pores is much larger than the area of the outer surface of the porous structure, the above expression is used here for convenience.

In the case of the porous structure made of calcium phosphate ceramics, it preferably has a high porosity, specifically a porosity of as much as 60-95%, when much bone should be formed in the porous structure without necessity of having high strength. On the other hand, when the porous structure should have enough strength, its porosity is preferably 40-60%.

### (2) Granular porous structure

In the case of the granular porous structure, it is desirable that bone is well formed in and between the granules in a portion in which the porous structure is implanted. Though the granular porous bone structure (filler) preferably has a structure having fine communicating pores having an average diameter of 0.005-50 µm throughout its entire body, the fine communicating pores being open on an outer surface of said porous structure at a density of 1 or more per an area of 50 µm x 50 µm, it may be obtained by pulverizing a porous structure having communicating macro-pores.

### (a) Granular porous bone structure having fine communicating pores

The granules preferably have an average diameter of 1 mm or less. In view of the fact that the formation of bone occurs mainly on granule surfaces but is not so expected in the granules, granules of more than 1 mm provide too wide gaps from the aspect of forming bone throughout gaps between the implanted granules and the implanted region, resulting in little bone formation leaving more granules free from formed bone. On the other hand, when the average diameter of the granules is less than 100 µm, there are too narrow gaps between the granules, making it likely that the intrusion and formation of blood vessels supplying nutrition are retarded. Accordingly, the average diameter of the granules is preferably 100 µm to 1 mm.

### (b) Granules obtained by pulverizing porous structure having communicating macro-pores

Because these granules have communicating macro-pores and fine communicating pores, bone is formed even in the granules of more than 1 mm. Accordingly, the average diameter of these granules is not so restrictive unlike the granules in (a) above. For instance, when a mixture of granules of more than 1 mm and granules of 1 mm or less is implanted in bone defects or cavities, good bone formation occurs in the entire implanted region. Accordingly, the above granules obtained by pulverizing the porous structure having communicating macro-pores may have an average diameter ranging from 100 µm to several millimeters.

### (B) Structure, only surface layer of which is porous

The structure, only a surface layer of which is porous, (simply called "surface-porous structure") includes (1) a structure whose porous surface layer has an average thickness of less than 100 µm, and (2) a structure whose porous surface layer has an average thickness of 100 µm or more. Both porous structures can be used as artificial bones or dental roots such as outer heads, etc. implanted in the bone.

When the entire structure is covered with a porous surface layer, a matrix of the surface-porous structure, a portion covered with the surface layer, may not be a biocompatible material. However, from the aspects of the integrity of the surface layer and the matrix and easy production of the surface-porous structure, both are preferably made of the same biocompatible material. In this case, any surface-porous structure comprises a matrix of a dense biocompatible material, and a porous layer integrally covering the matrix.

### (1) Structure whose porous surface layer has average thickness of less than 100 µm

The porous structure whose porous surface layer has an average thickness of less than 100 µm preferably has fine communicating pores having an average diameter of 0.005-50 µm at a density of 1 or more per a surface area of 50 µm x 50 µm as a whole. When this surface-porous structure whose porous surface layer is impregnated with fine bone powder is implanted as an artificial bone or dental root into the bone, the differentiation and formation of osteoblasts occurs spontaneously on a surface of the artificial bone or dental root, so that it is bonded to the surrounding bone surely and quickly. Because there is essentially a bone-forming environment around the artificial bone or dental root implanted into the bone, there is no need to artificially create an environment having bone-proliferating factors at high concentrations around the artificial bone or dental root.

### (2) Structure whose porous surface layer has an average thickness of 100 µm or more

This surface-porous structure preferably has a porous surface layer having an average thickness of 100 µm or more throughout the surface, with communicating macro-pores having an average diameter of 100-1000 µm, preferably 100-500 µm open at its outer surface, and fine communicating pores having an average diameter of 0.005-50 µm open in the communicating macro-pores. The communicating macro-pores are preferably open to the outer surface of said surface-porous structure at a density of 1 or more per an area of 1000 µm x 1000 µm, and the fine communicating pores are preferably open to the surface of the communicating macro-pores at a density of 1 or more per an area of 50 µm x 50 µm. In place of the fine communicating pores, the surface-porous structure may have fine recesses having an average diameter of 0.005-50 µm and an average depth of 0.005-50 µm, which are open in the communicating macro-pores at a density of 1 or more per an area of 50 µm x 50 µm.

When this surface-porous structure with a thick porous surface layer impregnated with fine bone powder is implanted as an artificial bone or dental root into the bone, strong bone is spontaneously formed inside and on the porous surface layer, and its bonding to the surrounding bone provides strong implant in the bone.

### [3] Surface-roughened structure

The surface-roughened structure of the present invention, whose dense matrix is surface-roughened, is used as an artificial bone or dental root such as an outer head, etc. implanted into the bone. The rough surface of the surface-roughened structure is preferably provided with fine recesses having an average diameter of 0.005-50 µm and an average depth of 0.005-50 µm at a density of 1 or more per an area of 50 µm x 50 µm. When the surface-roughened structure whose surface recesses are impregnated with fine bone powder is implanted into the bone as an artificial bone or dental root, spontaneous differentiation and formation of osteoblasts occurs on a surface of the artificial bone or dental root.

### [4] Production method

### (A) Production method of porous structure

### (1) Production of porous structure

The porous structure per se is known, and porous hydroxyapatite blocks, for instance, are commercially available as "APACERAM® ." Accordingly, the detailed explanation of the production method of the porous structure is not necessary. Simply stating, when the biocompatible material is a ceramic or metal, usual production methods of the porous sintered body may be utilized. When the biocompatible material is a polymer, it can be molded with a foaming agent to produce the porous structure.

### (2) Impregnation with fine bone powder

To impregnate the porous structure with fine bone powder, a method of using a suspension of bone powder is preferable. This method will be explained below.

### (a) Preparation of bone powder suspension

Bone powder or demineralized bone powder (simply called "bone powder") finely pulverized in a pulverizer or mortar is mixed with a physiological saline solution or a plasma or serum of a patient receiving an implanting treatment, and then dispersed by using a stirrer and ultrasonic sound. Proliferation/differentiation factors, antiviral agents, antibacterial agents, antibiotics, immune inhibitors, etc. contributing to bone formation may be added.

When the physiological saline solution is used, it is preferable to introduce a whole blood, plasma or serum of a patient receiving an implant treatment into the fine communicating pores and recesses of the bone-powder-impregnated, porous structure just when the porous structure is used, thereby replacing the physiological saline solution by the whole blood, plasma or serum of the patient. This method is suitable when the bone-powder-impregnated, porous structures are produced and stored in advance for large numbers of unidentified cases.

### (b) Impregnation with bone powder suspension

To impregnate the porous structure with a bone powder suspension, it is preferable to use a vibrator, a vacuum apparatus, a stirrer, ultrasonic sound, etc. during immersion. With a vibrator in contact with a vessel containing the porous structure, the bone powder suspension is slowly dropped into the vessel. A porous structure having a thickness of 5 mm or more is preferably provided with penetrating pores in advance. A porous structure having a thickness of 10 mm or more is preferably provided with a non-penetrating hole, to which a syringe needle or a pipe is mounted, such that the dropped bone powder suspension can be sucked into the porous structure by an evacuating means such as a syringe, etc.

Residual air bubbles are then removed from the porous structure by an evacuation apparatus such as an oil-less vacuum pump, etc., and the pressure is returned to an atmospheric level to accelerate impregnation with the bone powder suspension. Using a stirrer, the agglomeration and precipitation of the bone powder are prevented. The vessel is then immersed in an ultrasonic sound vessel to disperse fine bone powder in the porous structure, thereby accelerating the fine bone powder to enter into the fine communicating pores or recesses of the porous structure.

### (c) Storage of bone-powder-impregnated, porous structure

Though the bone-powder-impregnated, porous structure is preferably transplanted or implanted immediately after the impregnation, it may be stored in a usual refrigerator in several days. It is preferable to use a freezer of -80°C or lower for storage for a longer period, and such freezer enables storage for several months.

### (B) Production method of surface-roughened structure

### (1) Production of surface-roughened structure

When the biocompatible material is a metal, it is possible to form fine recesses having an average diameter of 0.005-50 µm and an average depth of 0.005-50 µm on its surface by an aqua regia treatment, an anodizing treatment, or their combinations with a grid-blasting treatment, etc. Also, when the biocompatible material is a calcium phosphate ceramic, recesses can be formed by a grid-blasting treatment and/or an acid treatment. However, the production of the surface-roughened structure is not restricted to these methods.

### (2) Impregnation with fine bone powder

To impregnate the recesses of the surface-roughened structure with fine bone powder, an impregnation method with a bone powder suspension is preferably used like in the case of the porous structure. Accordingly, the explanation in the section of "(2) Impregnation with fine bone powder" with respect to the porous structure is applicable to the impregnation of the surface-roughened structure with fine bone powder as it is.

The present invention will be explained in further detail referring to Examples below without intention of restricting the present invention thereto.

### Example 1

Bone pieces were taken from the tibia of an adult dog by a trepan bar mounted to an electric dental engine, and pulverized in a physiological saline solution in an alumina mortar in a sterile environment. After stirring a physiological saline solution containing the resultant fine bone powder, whose size was mostly 50 µm or less and predominantly in the order of sub-microns, thereby having an average diameter of 50 µm or less, 14 cc of the physiological saline solution containing fine bone powder was introduced into a 15-cc-conical tube, which was rotated at a speed reaching 3000 rpm by a centrifugal separator. The centrifugal separator was braked when the speed reached 3000 rpm, and the physiological saline solution was removed. The precipitated fine bone powder was mixed with a plasma of the same adult dog such that its volume became 10-30 times (about 80-240 times the volume of dense bone), to prepare a bone powder suspension.

The bone powder suspension was dropped into a vessel containing a porous hydroxyapatite structure (porosity: about 85%) of about 6 mm x 6 mm x 6 mm having communicating macro-pores mostly having an average diameter within 100-500 µm and fine communicating pores mostly having an average diameter of 50 µm or less, while shaking the vessel by a vibrator. The porous structure was not provided with penetrating pores. Using an ultrasonic stirrer of 100 W and 28 kHz, an impregnating treatment was conducted at a temperature of 10°C or lower for 10 minutes. After impregnated with the fine bone powder, 1 unit of human thrombin was added per 1 cc of the plasma for blood coagulation.

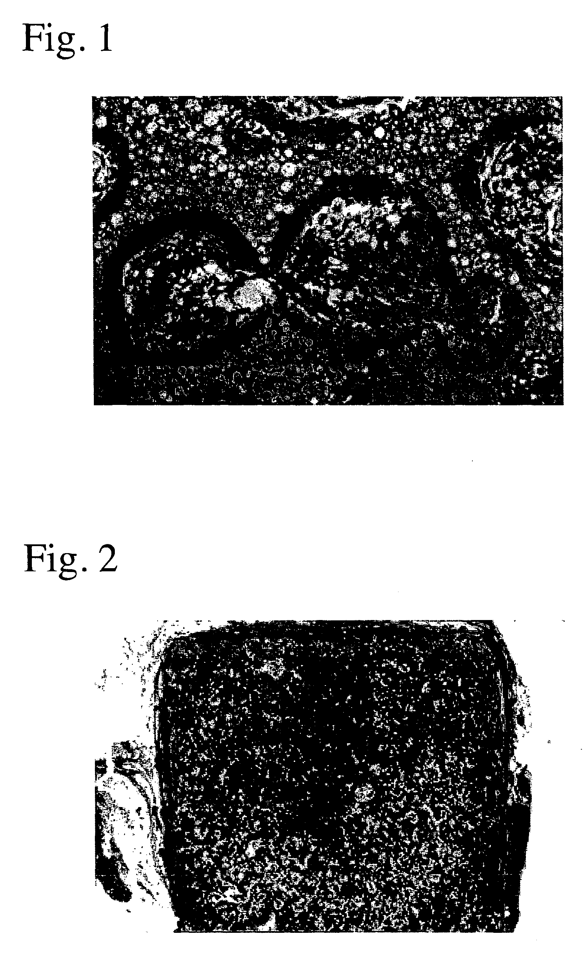

The resultant bone-powder-impregnated, porous structure was hypodermically transplanted into a fat tissue of the back of the same dog. After 2, 4, 6 and 8 weeks, respectively, passed from the transplantation, the bone-powder-impregnated, porous structure was extracted together with a surrounding fat tissue, to observe how a bone tissue was formed in the bone-powder-impregnated, porous structure. At a time when 2 weeks passed from the transplantation, blood components remained in a wide region of the bone-powder-impregnated, porous structure, with no bone formation observed even in the surrounding tissue. However, at a time when 4 weeks passed, most bone powder entering into the communicating macro-pores of the bone-powder-impregnated, porous structure already disappeared by absorption, with bone formation observed on the communicating macro-pores (Fig. 1). Further, at a time when 8 weeks passed, bone was formed in the entire bone-powder-impregnated, porous structure, and sinusoidal capillaries and a blood-producing bone marrow were formed in centers of the communicating macro-pores, indicating that the porous structure was filled with normal bone tissues. Also observed was a usual fat tissue as a transplant bed around the porous structure (Fig. 2).

### Example 2

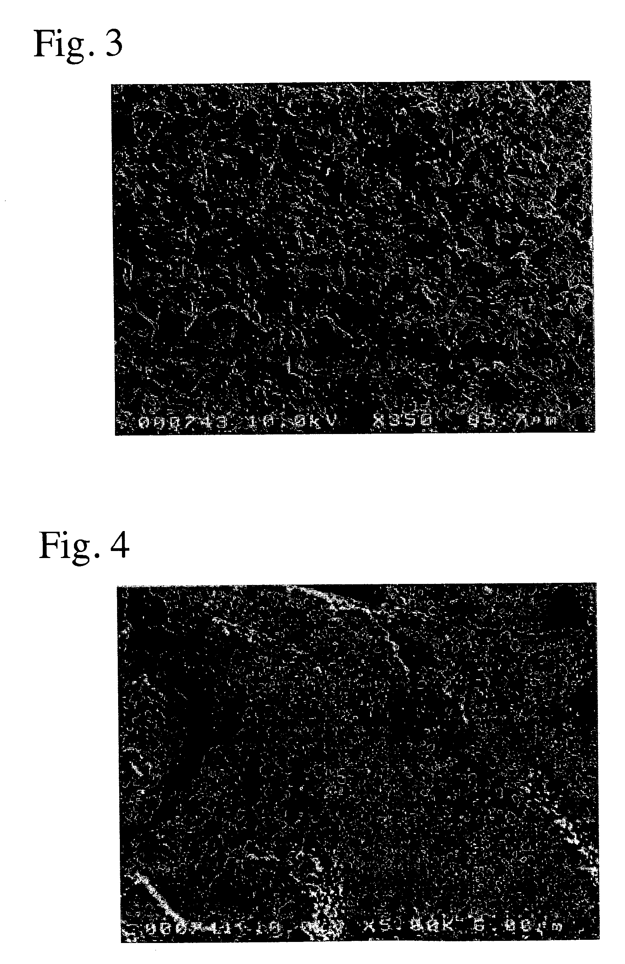

An artificial dental root formed by dense, sintered hydroxyapatite ("APACERAM® " available from Pentax Corp.) was blasted with 50-µm alumina beads at 2 atms to provide the entire surface of the artificial dental root with recesses having an average diameter of about 10 µm and an average depth of about 5 µm (Fig. 3). Further, ultrasonic sound of 100 W and 28 kHz was irradiated to the artificial dental root in an aqueous tetracycline hydrochloride solution at pH of about 2.5 to conduct etching for 5 minutes, thereby forming recesses between primary particles of sintered hydroxyapatite having diameters of about 0.5 µm (Fig. 4). Thus obtained was a porous structure having a so-called "biphasic rough surface," which had two types of recesses with different average diameters and average depths.

A rough surface of a surface-roughened, artificial dental root of hydroxyapatite was impregnated with fine bone powder in the same manner as in Example 1. After the impregnation of the fine bone powder, the artificial dental root was implanted to the ischium of an adult dog. The ischium including the artificial dental root was extracted after 4 weeks, to observe how bone was formed on a surface of the artificial dental root.

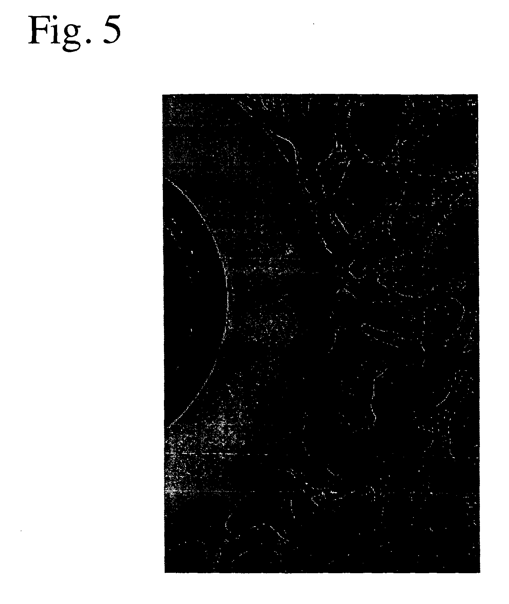

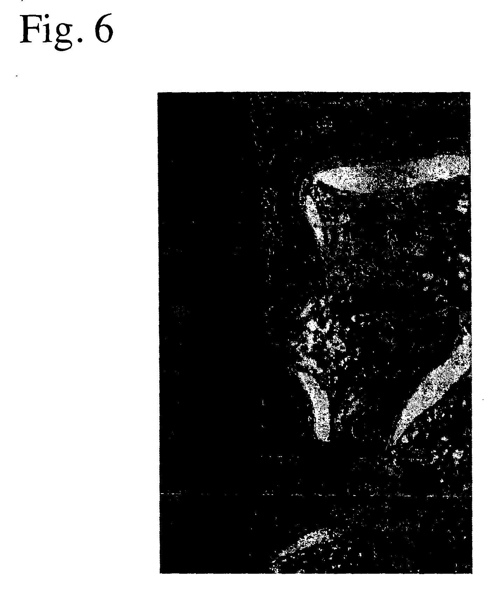

It was thus found that bone was formed on substantially the entire surface of the bone-powder-impregnated, surface-roughened, artificial dental root of hydroxyapatite (Fig. 5), and that the bone was directly bonded to the rough surface of hydroxyapatite (Fig. 6). Bone was formed more on the bone-powder-impregnated, surface-roughened, artificial dental root of hydroxyapatite than on the artificial dental root of hydroxyapatite not impregnated with bone powder.

### Example 3

An artificial dental root of titanium "TiUnite" available from Nobel Biocare of Sweden was prepared as an artificial dental root of titanium. This artificial dental root of titanium has an anodized layer (porous TiO₂ layer) as thick as 10-1 µm on a surface, and the porous layer has an average pore size of 10 µm or less (peak pore size: 1-2 µm), and an average surface roughness of 1.2 µm.

A porous surface layer of the surface-porous, artificial dental root of titanium was impregnated with fine bone powder in the same manner as in Example 1. After the impregnation of the fine bone powder, the artificial dental root was implanted to the ischium of an adult dog. The ischium including the artificial dental root was extracted after 4 weeks, to observe how bone was formed on a surface of the artificial dental root.

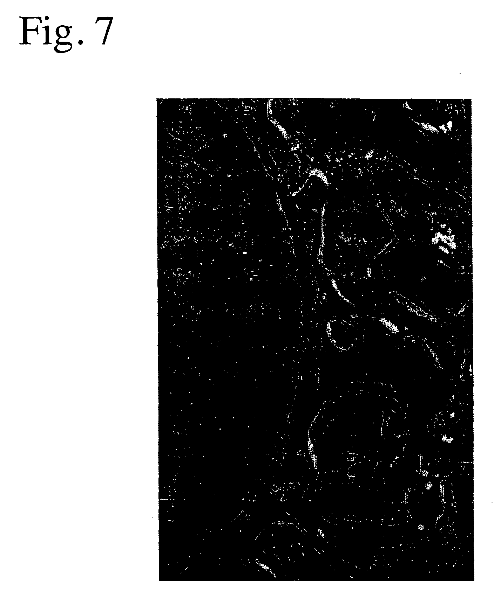

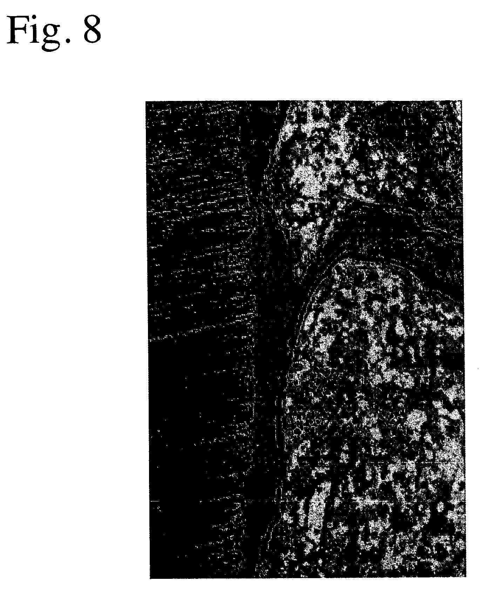

As a result, it was observed that bone was formed on a titanium oxide layer of the bone-powder-impregnated, surface-porous, artificial dental root of titanium in a wide region (Fig. 7), and that there was substantially no uncalcified layer observed between the rough titanium oxide layer surface and the bone on an optical-microscopic level (Fig. 8). A bone formation ratio was much higher on the bone-powder-impregnated, surface-porous, artificial dental root of titanium than on the surface-porous, artificial dental root of titanium not impregnated with bone powder (bone formation ratio: about 50%).

### INDUSTRIAL APPLICABILITY

The bone-powder-impregnated, porous or surface-roughened structure of the present invention has fine communicating pores and/or recesses, or communicating macro-pores and fine communicating pores and/or recesses, into which a small amount of fine bone powder has entered, so that the fine bone powder can exhibit its potential bone-forming capability as much as possible. Thus, the bone-powder-impregnated, porous structure of the present invention can regenerate a large amount of bone in a short period of time. Even in the case of the use of autologous bone, which is accompanied by invasion, bone can be regenerated in an amount of several tens of times or more of the autologous bone, as effective as fully compensating the disadvantages of the invasion.

The bone-powder-impregnated, porous structure of the present invention can be used safely in wide varieties of applications, because the regeneration of bone occurs locally not only inside or on the bone but also in a hypodermic fat tissue, etc. in a human body. The bone-powder-impregnated, porous structure of the present invention having such features is suitable as an artificial bone for bone regeneration.

The bone-powder-impregnated, surface-roughened structure and the porous structure having a surface layer impregnated with bone powder according to the present invention can be used as artificial bones and dental roots such as outer heads, etc., which have excellent ability to bond to surrounding bones, because they have spontaneous bone-forming capability on their surfaces.

## Claims

1. A bone-powder-impregnated, porous structure comprising a porous matrix made of a biocompatible material impregnated with fine bone powder.

2. The bone-powder-impregnated, porous structure according to claim 1, wherein it has fine communicating pores having an average diameter of 0.005-50 µm in its entire body, said fine communicating pores being open on an outer surface of said porous structure at a density of 1 or more per an area of 50 µm x 50 µm.

3. The bone-powder-impregnated, porous structure according to claim 1, wherein it has communicating macro-pores having an average diameter of 100-1000 µm in its entire body, which are open on an outer surface of said porous structure at a density of 1 or more per an area of 1000 µm x 1000 µm, and fine communicating pores having an average diameter of 0.005-50 µm, which are open on inner walls of said communicating macro-pores at a density of 1 or more per an area of 50 µm x 50 µm.

4. The bone-powder-impregnated, porous structure according to claim 1, wherein it has communicating macro-pores having an average diameter of 100-1000 µm in its entire body, which are open on an outer surface of said porous structure at a density of 1 or more per an area of 1000 µm x 1000 µm, and fine recesses having an average diameter of 0.005-50 µm and an average depth of 0.005-50 µm, which are open on inner walls of said communicating macro-pores at a density of 1 or more per an area of 50 µm x 50 µm.

5. The bone-powder-impregnated, porous structure according to any one of claims 1-4, wherein said biocompatible material is at least one selected from the group consisting of ceramics, metals, and polymers.

6. The bone-powder-impregnated, porous structure according to claim 5, wherein said ceramics are calcium phosphate ceramics.

7. The bone-powder-impregnated, porous structure according to any one of claims 1-6, wherein said fine bone powder is obtained by pulverizing living bone.

8. The bone-powder-impregnated, porous structure according to any one of claims 1-6, wherein said fine bone powder is demineralized bone powder.

9. The bone-powder-impregnated, porous structure according to any one of claims 1-8, wherein said fine bone powder has an average diameter of 50 µm or less.

10. The bone-powder-impregnated, porous structure according to any one of claims 1-9, wherein the entire structure is porous.

11. The bone-powder-impregnated, porous structure according to any one of claims 1-9, wherein only a surface layer of said structure is porous.

12. A method for producing the bone-powder-impregnated, porous structure recited in any one of claims 1-11, comprising the steps of preparing said fine bone powder, and impregnating said porous structure with said fine bone powder.

13. The method for producing a bone-powder-impregnated, porous structure according to claim 12, wherein said porous structure is impregnated with fine bone powder in the form of a suspension.

14. An artificial bone comprising the bone-powder-impregnated, porous structure recited in claim 10.

15. An artificial bone comprising the bone-powder-impregnated, porous structure recited in claim 11.

16. An artificial dental root comprising the bone-powder-impregnated, porous structure recited in claim 11

17. A bone-powder-impregnated, surface-roughened structure comprising a surface-roughened matrix made of a biocompatible material, which is impregnated with fine bone powder.

18. The bone-powder-impregnated, surface-roughened structure according to claim 17, wherein said surface-roughened structure has fine recesses having an average diameter of 0.005-50 µm and an average depth of 0.005-50 µm, which are open on its entire outer surface at a density of 1 or more per an area of 50 µm x 50 µm.

19. The bone-powder-impregnated, surface-roughened structure according to claim 17 or 18, wherein said biocompatible material is at least one selected from the group consisting of ceramics, metals, and polymers.

20. The bone-powder-impregnated, surface-roughened structure according to any one of claims 17-19, wherein said fine bone powder is obtained by pulverizing living bone.

21. The bone-powder-impregnated, surface-roughened structure according to any one of claims 17-19, wherein said fine bone powder is demineralized bone powder.

22. The bone-powder-impregnated, surface-roughened structure according to any one of claims 17-21, wherein said fine bone powder has an average diameter of 50 µm or less.

23. A method for producing the bone-powder-impregnated, surface-roughened structure recited in any one of claims 17-22, comprising the steps of preparing said fine bone powder, and impregnating said surface-roughened structure with said fine bone powder.

24. The method for producing a bone-powder-impregnated, surface-roughened structure according to claim 23, wherein a rough surface of said surface-roughened structure is impregnated with fine bone powder in the form of a suspension.

25. An artificial bone comprising the bone-powder-impregnated, surface-roughened structure recited in any one of claims 17-22.

26. An artificial dental root comprising the bone-powder-impregnated, surface-roughened structure recited in any one of claims 17-22.
